(19) **Europäisches Patentamt / European Patent Office / Office européen des brevets**

(11) **EP 3 782 656 A1**

(12) **EUROPEAN PATENT APPLICATION**
published in accordance with Art. 153(4) EPC

(43) Date of publication:
**24.02.2021 Bulletin 2021/08**

(21) Application number: **19787618.8**

(22) Date of filing: **17.04.2019**

(51) Int Cl.:
**A61L 15/42** (2006.01)　　**A61L 15/28** (2006.01)
**A61F 13/00** (2006.01)　　**A61F 13/36** (2006.01)
**C08L 5/08** (2006.01)　　**A61L 15/54** (2006.01)

(86) International application number:
**PCT/KR2019/004614**

(87) International publication number:
**WO 2019/203556 (24.10.2019 Gazette 2019/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.04.2018 KR 20180045193**

(71) Applicants:
• **Endovision Co., Ltd.**
**Daegu 41061 (KR)**

• **Jung, Min Ho**
**Daegu 42758 (KR)**

(72) Inventors:
• **KIM, Jonggeun**
**Daegu 42427 (KR)**
• **JUNG, Min Ho**
**Daegu 42758 (KR)**

(74) Representative: **Viering, Jentschura & Partner mbB**
**Patent- und Rechtsanwälte**
**Am Brauhaus 8**
**01099 Dresden (DE)**

(54) **MEDICAL DRESSING MATERIAL HAVING CHITOSAN FABRIC SPONGE STRUCTURE AND MANUFACTURING METHOD THEREFOR**

(57)　　A medical dressing material having a chitosan fabric sponge structure according to the present disclosure has a structure in which chitosan fibers derived from a chitosan fabric are located on a porous structure formed using freeze-drying. Therefore, the medical dressing material has an excellent hemostatic effect and exudate-absorbing ability while exhibiting a strong tensile force, is convenient to use, and is capable of being molded into various forms so as to be widely used for wounds by an edged thing, scalds, pressure ulcers, diabetes-related foot disorders, or various surgical areas in various body tissue parts such as the spine, brain, eye, ear, nose, and cervix.

FIG. 3A

FIG. 3B

## Description

### Technical Field

The present disclosure relates to a medical dressing material having a chitosan fabric sponge structure and a method of manufacturing the same. More particularly, the present disclosure relates to a medical dressing material having a chitosan fabric sponge structure, which is manufactured using a process that includes acid-treating a chitosan fabric to perform gelation, followed by freeze drying, thereby exhibiting excellent effects, and to a method of manufacturing the same.

### Background Art

When trauma to the body occurs, blood clotting may be achieved based on a blood-clotting mechanism with only simple measures if the wound is not deep. However, when the wound is deep or tissue is removed for surgery or examination, it is critically required to provide artificial hemostasis or dressing on the affected area in order to prevent excessive bleeding.

In this regard, chitosan is a compound obtained by deacetylating chitin contained in the cell walls of crabs, shrimp exoskeleton, squid skeleton, and microorganisms such as fungi and bacteria. Chitosan has been discovered to have excellent biocompatibility and promote wound healing, and thus has attracted attention as a medical material since the 1990s. Currently, chitosan is widely used in wound-healing agents, artificial skin, embolic materials, blood coagulants, artificial kidney membranes, biodegradable surgical suture threads, and antimicrobial materials. Chitosan is used for hemostasis and dressing, and many studies are being conducted on the use thereof to quickly heal wounds.

In a number of conventional techniques, chitosan has been processed into non-woven fabrics for use in treating wounds such as scalds, cuts, and wounds by an edged thing. For example, Korean Laid-Open Patent Application No. 2001-0047248 relates to a method of manufacturing a medical chitosan nonwoven fabric. However, such a non-woven fabric dressing material has problems in that the wound is maintained in a dry state, thus slowing treatment, the dressing material is attached to the wound surface, which makes exchange thereof difficult, and damage to new tissues occurs and pain is experienced in a process of removing the dressing material. Further, there is the inconvenience of having to change the dressing material several times a day because a large amount of exudates is generated in the initial stage of healing.

Accordingly, there has been an effort to solve this by processing chitosan into a sponge form. For example, Korean Patent No. 0644369 relates to a chitosan-gelatin-algin sponge for skin dressing and a method of manufacturing the same, and provides a method of forming a sponge structure using a wound dressing for external damage to the skin, thus improving antimicrobial activity and moisturizing power. However, this sponge structure has weak tensile force due to the structural characteristics thereof, so the mechanical properties thereof are poor. Accordingly, there are problems in that the sponge is easy to damage during use, creating a large amount of debris, and that it is not easy to remove the sponge because the sponge is easily crushed during a process of removing the sponge.

Therefore, in order to solve the problems, there is a need for a technique for providing effective hemostasis and dressing for the affected area, thus enabling quick healing of a wound.

### Disclosure

### Technical Problem

An objective of the present disclosure is to solve the problems of the conventional technology as described above and technical tasks that have been previously requested.

Specifically, an objective of the present disclosure is to provide a medical dressing material using chitosan, which has an excellent hemostatic effect and exudate-absorbing ability, and moreover exhibits strong tensile force, is convenient to use, and creates a humid environment in the wound area to thus exhibit an excellent therapeutic effect, and also to provide a method of manufacturing the same.

### Technical Solution

The present disclosure provides a method of manufacturing a medical dressing material having a chitosan fabric sponge structure. The method includes

    (A) preparing a chitosan fabric,
    (B) immersing the chitosan fabric in an acidic organic solution, thus performing acid treatment,

(C) washing the acid-treated chitosan fabric using alcohol,

(D) agitating a gel obtained by mixing the washed chitosan fabric with an aqueous solvent, thus performing homogenization, and

(E) freeze-drying the homogenized chitosan fabric gel to manufacture a chitosan fabric sponge.

In step (B), the acid treatment may be performed for 1 to 10 minutes.

In step (B), an acidic compound and an alcohol solvent may be mixed at a ratio of 1 : 99 to 7 : 93 in the acidic organic solution based on the weight thereof.

The acidic compound may be one or more selected from the group consisting of hydrochloric acid, acetic acid, ascorbic acid, nitric acid, sulfuric acid, hydrofluoric acid, and phosphoric acid.

The alcohol solvent may be one or more selected from the group consisting of methanol, ethanol, propanol, and butanol.

In step (C), the washing may include a first washing step, a second washing step, and a third washing step, continuously performed using 100% alcohol.

Each of the first washing step, the second washing step, and the third washing step may be performed for 20 to 90 seconds.

In step (D), the aqueous solvent may be distilled water or ultrapure water.

In step (D), the gel may be obtained by mixing 1 to 5 parts by weight of the washed chitosan fabric based on 100 parts by weight of the aqueous solvent.

In step (D), the homogenization may be performed at 5,500 to 20,000 rpm for 1 to 10 minutes using a homogenizer.

In detail, step (E) may include

(E-1) injecting the homogenized chitosan fabric gel into a molding frame, and

(E-2) putting the molding frame into a freeze dryer to perform freeze-drying, thus manufacturing the chitosan fabric sponge.

In step (E-2), the freeze-drying may be performed while the temperature is increased from a first temperature ($\Delta t_1$) to a second temperature ($\Delta t_2$) for 2.5 to 5.5 days, the first temperature ($\Delta t_1$) may be -40 to -50°C, and the second temperature ($\Delta t_2$) may be 40 to 50°C.

A preliminary freezing step may be performed between steps (E-1) and (E-2), and the preliminary freezing step may include

(E-1a) putting the molding frame into the freeze dryer and storing the molding frame at 3 to 5°C for 450 to 550 minutes to thus remove air bubbles, followed by cooling to 0°C,

(E-1b) maintaining a temperature for a first time ($\Delta m_1$) after step (E-1a),

(E-1c) reducing the temperature by $\Delta T$ for a second time ($\Delta m_2$) after step (E-1b), and

(E-1d) repeating the step (E-1b) and step (E-1c) until the temperature becomes -40 to -50°C after the step (E-1c), and then maintaining the temperature for 100 to 140 minutes.

Each of the first time ($\Delta m_1$) and the second time ($\Delta m_2$) may be 20 to 70 minutes, and $\Delta T$ may be 5 to 10°C.

In the present disclosure, the method may further include

(F) performing thermal compression on the freeze-dried chitosan fabric sponge.

In the present disclosure, the method may further include

after step (E) or step (F),

(G) adding at least one X-ray sensitive material so as to identify the location of the medical dressing material using an X-ray device.

The present disclosure provides

a medical dressing material having a chitosan fabric sponge structure in which chitosan macro fibers and chitosan micro fibers derived from a chitosan fabric are located on a porous structure formed using freeze-drying.

The medical dressing material may have a density of 0.01 to 0.1 g/cm$^3$.

The medical dressing material may have a wet tensile strength of 500 to 1500 gf and a dry tensile strength of 1000 to 2000 gf.

The medical dressing material may include at least one X-ray sensitive material so as to identify the location of the dressing material using an X-ray device.

The medical dressing material may include a hemostatic dressing material and a dressing material for wounds by an edged thing.

**Advantageous Effects**

A medical dressing material having a chitosan fabric sponge structure according to the present disclosure has a structure in which chitosan fibers derived from a chitosan fabric are located on a porous structure formed using freeze-drying. The medical dressing material is provided as an intermediate form between a sponge and a fabric, and has all of the advantages of both.

That is, excellent absorption ability and expansion power are exhibited due to the porous structure, and high tensile force is secured due to the fabric structure. Accordingly, since the medical dressing material has excellent ability to withstand moisture and friction, it is possible to minimize damage to the product in use and secure excellent ease of use. Due to these structural characteristics, the medical dressing material having the chitosan fabric sponge structure according to the present disclosure exhibits high blood coagulation ability and a high blood absorption rate. Therefore, the hemostatic effect and exudate absorption management ability are excellent, which is advantageous in creating a humid environment in the wound area, so an excellent therapeutic effect is secured.

Further, the medical dressing material having the chitosan fabric sponge structure according to the present disclosure is manufactured using a process that includes acid-treating a chitosan fabric to perform gelation, followed by freeze drying. Accordingly, the medical dressing material is not only capable of being molded in various forms during the freeze drying process, but also has an excellent deodorization rate and antibacterial effect due to the unique effect of the chitosan fabric.

Therefore, the medical dressing material having the chitosan fabric sponge structure according to the present disclosure is capable of being widely used for wounds by an edged thing, scalds, pressure ulcers, diabetes-related foot disorders, or various surgical areas in various body tissue parts, such as the spine, brain, eye, ear, nose, and cervix.

**Description of Drawings**

[0034]

　　FIGS. 1A and 1B are a SEM image of Comparative Example 1 and a mimetic view thereof, respectively;
　　FIGS. 2A and 2B are a SEM image of Comparative Example 2 and a mimetic view thereof, respectively;
　　FIG. 3A is a skin SEM image and a cross-section SEM image of Example 1, and FIG. 3B is a skin SEM image and a cross-section SEM image of Example 2;
　　FIG. 4 is a mimetic view of SEM images of Examples 1 and 2;
　　FIG. 5 is a view showing a blood clotting evaluation method;
　　FIGS. 6A, 6B and 6C are graphs and photographs showing the results of blood clotting evaluations of Example 1 and Comparative Examples 1, 3, 4, and 5;
　　FIG. 7 is a graph showing the results of deodorization rate evaluations of Example 1 and Comparative Examples 4 and 5;
　　FIG. 8 is a photograph showing an antibacterial effect evaluation method;
　　FIG. 9 is a photograph showing the results of the antibacterial effect evaluations of Example 1 and Comparative Examples 4 and 5;
　　FIGS. 10A and 10B are photographs showing Example 1, which is commercialized; and
　　FIGS. 11A, 11B and 11C are photographs showing Example 2, which is commercialized.

**Best Mode**

As described above, chitosan is excellent in biocompatibility and has a wound-healing promotion effect, so chitosan is used in various ways as a dressing material and has been conventionally processed into a non-woven fabric or sponge form for use.

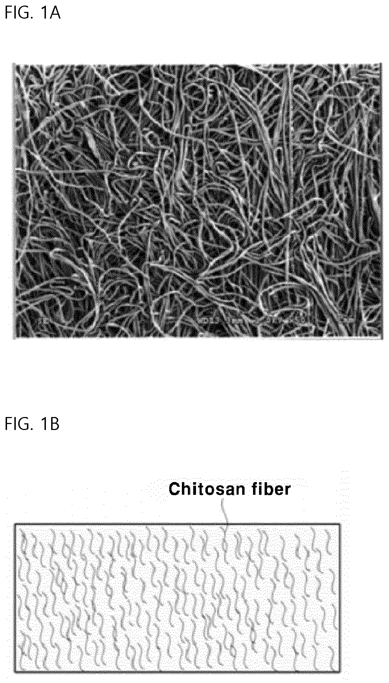

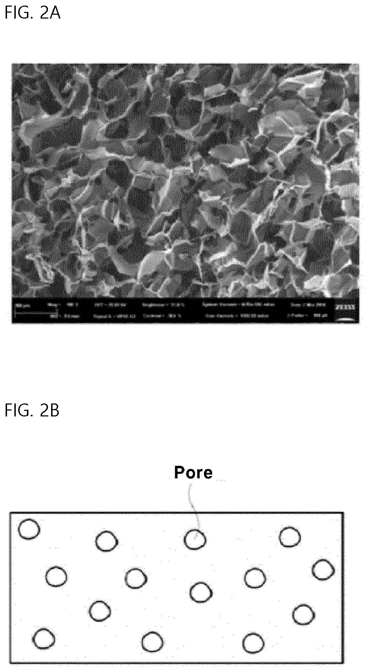

The following FIGS. 1A, 1B, 2A, and 2B are SEM images and mimetic views of a dressing material using chitosan processed according to a conventional technology. Specifically, FIGS. 1A and 1B are a SEM image of the dressing material processed into a non-woven fabric type and a mimetic view thereof. FIGS. 2A and 2B are a SEM image of the dressing material processed into a porous sponge structure and a mimetic view thereof.

However, in the case of the non-woven fabric type of FIGS. 1A and 1B, due to the structural characteristics thereof, there is a limit in the amount of exudates that are absorbed, and a dry environment is created around the wound, so treatment may be delayed. Further, since the non-woven fabric type easily becomes attached to the wound surface, damage to new tissue and pain occur in the removal process thereof.

In the case of the sponge structure of FIGS. 2A and 2B, tensile force is weak due to structural characteristics thereof, so the mechanical properties thereof are relatively poorer than those of the non-woven fabric type. Accordingly, there are problems in that the sponge is easily damaged during use, creating a large amount of debris, and in that the sponge is easily crushed during a process of removing the sponge.

Therefore, the present disclosure provides a medical dressing material having all the merits of the dressing material having the above-described structural characteristics while overcoming the drawbacks of the non-woven fabric-type dressing material and the sponge-type dressing material according to the conventional technology.

Specifically, the present disclosure provides a method of manufacturing a medical dressing material having a chitosan fabric sponge structure. The method includes

(A) preparing a chitosan fabric,
(B) immersing the chitosan fabric in an acidic organic solution, thus performing acid treatment,
(C) washing the acid-treated chitosan fabric using alcohol,
(D) agitating a gel obtained by mixing the washed chitosan fabric with an aqueous solvent, thus performing homogenization, and
(E) freeze-drying a homogenized chitosan fabric gel to manufacture a chitosan fabric sponge.

The medical dressing material having the chitosan fabric sponge structure according to the present disclosure is manufactured using a process that includes acid-treating a chitosan fabric to perform gelation, followed by freeze drying. Accordingly, due to the structure in which chitosan fibers derived from a chitosan fabric are located on a porous structure formed using freeze-drying, the medical dressing material is provided as an intermediate form between a sponge and fabric, and has all of the advantages of both.

That is, as can be seen in the following Experimental Examples, the medical dressing material exhibits excellent absorption ability and expansion power due to the porous structure thereof, and also a high tensile strength due to the fabric structure. Accordingly, the medical dressing material has excellent ability to withstand moisture and friction, thus minimizing damage to products during the use thereof, and is convenient to use. Due to the above-described structural characteristics, the medical dressing material exhibits high blood coagulation ability and a high blood absorption rate. Therefore, the hemostatic effect and exudate absorption management ability are excellent, which is advantageous in creating a humid environment in the wound area, so an excellent therapeutic effect is secured.

In detail, in the step (A), the chitosan fabric may be in the form of chitosan yarns or chitosan non-woven fabrics. The manufacturing method thereof and method of obtaining the same are known in the art, and thus a detailed description thereof will be omitted.

In the step (B), the amine group ($NH_2$) of the chitosan molecule may be converted into $-NH_3^+$ through acid treatment of the chitosan fabric. In this way, the acid-treated chitosan fabric not only facilitates gelation, but also adsorbs platelets to induce blood clotting, thereby promoting wound healing and having improved absorption ability.

The acid treatment step, that is, immersion, may be performed for an immersion time of 1 to 10 minutes, and the acidic compound and the alcohol solvent may be mixed at a ratio of 1 : 99 to 7 : 93 in the acidic organic solution based on the weight thereof.

When the acid treatment time is less than 1 minute, or when the weight ratio of the acidic compound is less than 1 or the weight ratio of the alcohol solvent is more than 99, since acid treatment is not sufficiently performed, it is difficult to accomplish gelation of the chitosan fabric. Further, when the acid treatment time is more than 10 minutes, or when the weight ratio of the acidic compound is more than 7 or the weight ratio of the alcohol solvent is less than 93, since the acid treatment is excessively performed, the physical properties of the chitosan fabric are deteriorated, and surface coating may be caused by excessive gelation. Accordingly, absorption ability may be reduced because it is difficult to absorb blood or exudates to the inside.

Thus, in detail, the acid treatment step may be performed for 2 to 8 minutes, and in more detail, may be performed for 3 to 5 minutes. Further, in detail, the acidic compound and the alcohol solvent may be mixed at a ratio of 2 : 98 to 4 : 96 in the acidic organic solution based on the weight thereof.

The acidic compound is not limited, as long as the compound is a compound exhibiting acidity, but examples thereof may be one or more selected from the group consisting of hydrochloric acid, acetic acid, ascorbic acid, nitric acid, sulfuric acid, hydrofluoric acid, and phosphoric acid. In detail, when one or more selected from hydrochloric acid, acetic acid, or ascorbic acid is used, the final product (medical dressing material) may be less odorous than when other acids are used. In more detail, the acidic compound may be hydrochloric acid.

The alcohol solvent is not limited, as long as the solvent dissolves acidic compounds, but examples thereof may be one or more selected from the group consisting of methanol, ethanol, propanol, and butanol. In detail, the alcohol solvent may be ethanol. The propanol and the butanol each include isomers thereof.

In the step (C), a washing step may be performed in order to remove the acidic organic solution component remaining in the acid-treated chitosan fabric.

The washing may include a first washing step, a second washing step, and a third washing step continuously performed using 100(vol)% alcohol. Each of the first washing step, the second washing step, and the third washing step may be performed for 20 to 90 seconds.

When the number of washings is less than 3 or when each washing time is less than 20 seconds, since washing may not be sufficiently performed, side reactions may occur during the subsequent gelation process or when the dressing material is in contact with the wound site due to the acidic component remaining in the chitosan yarns. When the number of washings is more than 3 or when the washing time is more than 90 seconds, the manufacturing process may be performed for an excessively lengthened period of time, and the acid-treated chitosan fabric may be physically and chemically damaged, which is not preferable. Accordingly, in detail, the washing may be performed for 30 to 60 seconds through the three individual steps.

The alcohol is 100(vol)% alcohol having strong volatility, and the same type of alcohol as in the acid treatment step may be used. In detail, the alcohol may be ethanol.

The washing method is not limited, but, for example, the acid-treated chitosan fabric and alcohol may be mixed with agitation to remove the acid, and pressure may be applied during this process. In the case of using a non-woven chitosan fabric, the washing may be performed by passing the non-woven chitosan fabric through an alcohol tank or simply immersing the non-woven chitosan fabric therein.

In the step (D), for the purpose of fine processing, the washed chitosan fabric may be mixed with the aqueous solvent to thus perform gelation, followed by homogenization.

The aqueous solvent may be, for example, distilled water or ultrapure water, and in detail, may be distilled water.

The gel may be obtained by mixing 1 to 5 parts by weight of the washed chitosan fabric based on 100 parts by weight of the aqueous solvent. When the content of the washed chitosan fabric is less than 1 part by weight based on 100 parts by weight of the aqueous solvent, since the content of the chitosan fabric is very low, there is a concern that the effect as a hemostatic dressing material may be reduced. When the content is more than 5 parts by weight, since it is not easy to perform gelation and the content of the aqueous solvent is relatively low, it may not be easy to create a moist environment in the wound, which is not preferable. Accordingly, in detail, 1 to 3 parts by weight of the washed chitosan fabric may be mixed based on 100 parts by weight of the aqueous solvent to manufacture a mixture in a gel state.

The homogenization serves to grind the mixture in a gel state in the homogenizer under friction caused by physical impact or pressure, so that chitosan particles are uniformly distributed in the mixture. When the chitosan particles are not uniformly distributed in the mixture, layers may be formed in the final product, which is not preferable.

A high-speed homogenizer or a high-pressure homogenizer may be used as the homogenizer, and in detail, a high-speed homogenizer may be used.

The homogenization may be performed at 5,500 to 20,000 rpm for 1 to 10 minutes. When the agitation speed and time are respectively slower and shorter than the above range, it is difficult to sufficiently perform homogenization. When the agitation speed and time are respectively very fast and long, efficiency is low in view of the manufacturing process and there is a concern that the gel-state mixture may be damaged, which is not preferable. Accordingly, in detail, the homogenization may be performed at 6,000 to 16,000 rpm for 3 to 7 minutes.

In step (E), in order to form pores by removing ethanol and moisture remaining in the homogenized chitosan fabric gel, freeze-drying may be performed.

Therefore, in detail, step (E) may include

(E-1) injecting the homogenized chitosan fabric gel into a molding frame, and
(E-2) putting the molding frame into a freeze dryer to perform freeze-drying, thus manufacturing the chitosan fabric sponge.

The freeze-drying is a method of freezing contents and then sublimating the contents into a gaseous vapor, without passing through a liquid state, in a vacuum, thereby accomplishing drying. In detail, in step (E-2), the freeze-drying may be performed while the temperature is increased from a first temperature ($\Delta t_1$) to a second temperature ($\Delta t_2$) for 2.5 to 5.5 days, the first temperature ($\Delta t_1$) may be -40 to -50°C, and the second temperature ($\Delta t_2$) may be 40 to 50°C.

When the freeze-drying time is less than 2.5 days or when the temperature is out of the above temperature range, remaining ethanol and moisture may not be sufficiently removed. When the freeze-drying time is more than 5.5 days, efficiency may be reduced in views of the manufacturing process, which is not preferable. Accordingly, in detail, the freeze-drying may be performed while the temperature is increased from -45°C to 45°C for 3 to 5 days.

In some cases, the preliminary freezing step may be included between the step (E-1) and the step (E-2), thereby further improving the absorption ability of the dressing material.

Therefore, the preliminary freezing step may include

(E-1a) putting the molding frame into a freeze dryer and storing the molding frame at 3 to 5°C for 450 to 550 minutes to thus remove air bubbles, followed by cooling to 0°C,
(E-1b) maintaining a temperature for a first time ($\Delta m_1$) after step (E-1a),
(E-1c) reducing the temperature by $\Delta T$ for a second time ($\Delta m_2$) after step (E-1b), and
(E-1c) repeating step (E-1b) and step (E-1c) until the temperature becomes -40 to -50°C after step (E-1c), and then maintaining the temperature for 100 to 140 minutes.

In detail, in step (E-1a), the molding frame may be put into the freeze dryer and then stored at 4°C for 500 minutes to thus remove air bubbles, followed by cooling to 0°C.

Each of the first time ($\Delta m_1$) and the second time ($\Delta m_2$) may be 20 to 70 minutes, and $\Delta T$ may be 5 to 10°C. In detail, each of the first time ($\Delta m_1$) and the second time ($\Delta m_2$) may be 30 to 60 minutes, and $\Delta T$ may be 5 to 10°C.

In step (E-1d), step (E-1b) and step (E-1c) may be repeated until the temperature becomes -45°C after step (E-1c), and the temperature may then be maintained for 100 to 140 minutes.

The above-described cooling temperature and temperature maintenance time ranges are optimal ranges for improving absorption ability without generating ice grains and inducing the formation of uniform pores during a subsequent heat treatment step. Temperatures and times respectively higher or lower than the temperature and time within this range are not preferable.

In some cases, the manufacturing method according to the present disclosure may further include

(F) performing thermal compression on the freeze-dried chitosan fabric sponge.

The freeze-dried chitosan fabric sponge may be directly applied to a wound. However, in some cases, thermal compression may be applied to suitably adjust the size and shape thereof, and expansion may be induced in application thereof, thereby improving the hemostatic effect and exudate absorption condition due to the compression. The thermal compression condition may be appropriately adjusted depending on the human body part to which the final product is applied. For example, when the medical dressing material is applied to the ears or nose, the medical dressing material may have the shape suitable for insertion into the body, and may have a flat pad shape when applied to other skin areas.

The thermal compression may be performed by applying pressure at 50 to 80°C for 1 to 20 seconds, and within the above ranges, temperature and time conditions may be appropriately adjusted depending on the final desired shape.

Further, in the present disclosure, in some cases, the method may further include

(G) adding at least one X-ray sensitive material so as to identify the location of the medical dressing material using an X-ray device.

In particular, when insertion thereof into the body is required in spinal, brain, and other organ surgery, an X-ray sensitive material may be included therein, thus being used to check the location of the medical dressing material. Further, after sufficient hemostasis is achieved, it may be easier to remove the medical dressing material.

The X-ray sensitive material is not limited, as long as the material is a material capable of sensing X-rays, but examples thereof may be a poly-crosslinked phthalocyanine compound. The method of adding the X-ray sensitive material is not limited, and the X-ray sensitive material may be appropriately added using a method known in the art, such as mixing with a raw material and attaching to a final material.

Further, the present disclosure provides a medical dressing material having a chitosan fabric sponge structure manufactured using the above-described method.

In detail, the present disclosure provides a medical dressing material in which chitosan macro fibers and chitosan micro fibers derived from a chitosan fabric are located on a porous structure formed using freeze-drying.

In detail, the medical dressing material may have a structure in which chitosan macro fibers having a diameter of several micrometers to tens of micrometers and chitosan micro fibers having a diameter of several nanometers are randomly arranged on a porous structure. Since moisture is removed during the freeze-drying process, the medical dressing material may include 100% chitosan.

As described above, the medical dressing material is provided as an intermediate form between a sponge and fabric, and has all of the advantages of both. That is, excellent absorption ability and expansion power are exhibited due to the porous structure, and high tensile force is secured due to the fabric structure. Accordingly, since the medical dressing material has excellent ability to withstand moisture and friction, it is possible to minimize damage to the product, such as the generation of debris, during use thereof, and the medical dressing material is convenient to use due to ease of removal thereof. Due to these structural characteristics, the medical dressing material exhibits high blood coagulation ability and a high blood absorption rate. Therefore, the hemostatic effect and exudate absorption management ability are excellent, which is advantageous in creating a humid environment in the wound area, so an excellent therapeutic effect is secured.

That is, the medical dressing material having the chitosan fabric sponge structure according to the present disclosure exhibits an excellent effect due to the synergy caused by the combination of the porous structure and the fabric structure. This is distinguished from a sponge structure having a porous structure according to the conventional technology and a non-woven fabric type including micropores formed due to agglomeration of chitosan fabrics according to the conventional technology.

In the present disclosure, the pores may have various shapes and sizes, and may exist in a continuous or discontinuous state from the outer surface of the dressing material to the interior thereof, and in some cases, the number of pores may be larger in the interior of the dressing material than in the outer surface thereof. Further, the average diameter and distribution form of chitosan fibers may depend on the type and shape of the chitosan fabric that is used as a raw material.

The medical dressing material may have a density of 0.01 to 0.1 $g/cm^3$. Further, the medical dressing material may be constituted so as to have a wet tensile strength of 500 to 1500 gf and a dry tensile strength of 1000 to 2000 gf. When the density, the wet tensile strength, or the dry tensile strength is excessively small outside the range defined above, the tensile force is poor, so the usage holding power is weak, and the medical dressing material is easily broken during the removal process. The case where the density, wet tensile strength, or dry tensile strength is excessively large is not preferable because the ability to absorb moisture and exudates may be lowered. Accordingly, in detail, the medical dressing material may be constituted so as to have a wet tensile strength of 1000 to 1500 gf and a dry tensile strength of 1500 to 2000 gf.

The medical dressing material may include at least one X-ray sensitive material so as to identify the location of the hemostatic dressing material using an X-ray device, and the X-ray sensitive material may be a poly-crosslinked phthalocyanine compound.

**Mode for Disclosure**

Hereinafter, Examples of the present disclosure will be described in detail. However, the following Examples are merely illustrative of the present disclosure, and the contents of the present disclosure are not limited to the following Examples.

Example 1

Chitosan yarns manufactured by Hismer company were prepared.

After the chitosan yarns were immersed in an acidic organic solution including 3 ml of hydrochloric acid and 97 ml of ethanol mixed therein for 3 minutes so as to be acid-treated, a washing process that was performed with 100% ethanol for 1 minute was repeated three times, thus removing the remaining acid.

After 2 g of the washed chitosan fabric was mixed with 100 ml of distilled water to manufacture a gel form, agitation was performed at 7000 rpm for 5 minutes using a high-speed homogenizer. Thereafter, the homogenized chitosan fabric gel was injected into a molding frame and then stabilized at 4°C for 10 hours (synchronization), followed by preliminary freezing under the conditions of Table 1 below.

[Table 1]

| Temperature (°C) | 4 | 0 | -10 | -10 | -20 | -20 | -30 | -40 | -45 | -45 |
|---|---|---|---|---|---|---|---|---|---|---|
| Section | Defoaming | Temperature is maintained | Temperature is reduced | Temperature is maintained | Temperature is reduced | Temperature is maintained | Temperature is reduced | Temperature is maintained | Temperature is reduced | Temperature is maintained |
| Time (minutes) | 500 | 60 | 60 | 30 | 60 | 30 | 30 | 30 | 30 | 120 |

EP 3 782 656 A1

Thereafter, freeze-drying was performed while the temperature was increased from -45°C to 45°C for 4 days, thus manufacturing a medical dressing material.

Example 2

Pressure was applied to the medical dressing material manufactured in Example 1 at 80°C for 10 seconds, thus manufacturing a pad-type medical dressing material.

Comparative Example 1

50 kg of a chitosan fabric was punched using a needle-punching or water-punching machine for 3 hours, thus manufacturing a dressing material in the form of a non-woven fabric.

Comparative Example 2

2 parts by weight of chitosan powder was mixed and agitated based on 100 parts by weight of distilled water to form a mixture in a gel state, and the same freeze-drying conditions as in Example 1 were applied to thus manufacture a dressing material in the form of a porous sponge.

Comparative Example 3

SURGICEL® manufactured by Johnson & Johnson Medical company was prepared as a dressing material.

Comparative Example 4

STANPAD™ manufactured by MANTIZ Logitech company was prepared as a dressing material.

Comparative Example 5

QuicClot® manufactured by Z-medica company was prepared as a dressing material.

Experimental Example 1

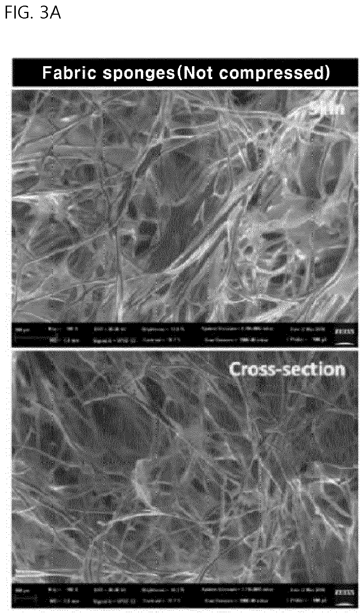

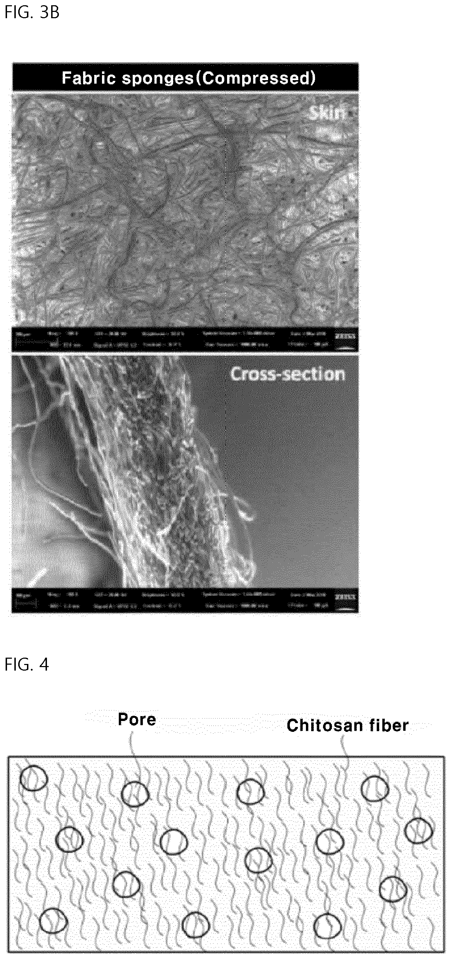

SEM images of the hemostatic dressing materials manufactured in Examples 1 and 2 and Comparative Examples 1 and 2 and mimetic views of the images are shown in the following FIGS. 1 to 4.
In detail, the following FIGS. 1A and 1B are a SEM image of a non-woven fabric-type dressing material of Comparative Example 1 and a mimetic view thereof, respectively. The following FIGS. 2A and 2B are a SEM image of the porous sponge-type dressing material of Comparative Example 2 and a mimetic view thereof, respectively. The following FIG. 3A is a skin SEM image and a cross-section SEM image of the medical dressing material of Example 1, and the following FIG. 3B is a skin SEM image and a cross-section SEM image of the medical dressing material of Example 2. The following FIG. 4 is a mimetic view of SEM images of Examples 1 and 2.
According to the following FIGS. 1 to 4, it can be confirmed that chitosan fibers derived from the chitosan fabric are located on the porous structure formed using freeze-drying in the case of the medical dressing materials of Examples 1 and 2 according to the present disclosure.

Experimental Example 2

The absorption ability, blood clotting, blood absorption rate, deodorization rate, ability to withstand moisture, ability to withstand friction, and antibacterial effect of hemostatic dressing materials according to the Examples and the Comparative Examples were evaluated.

2-1) Evaluation of absorption ability

(1) Test method

EN13726-1: Test methods for primary wound dressings. Aspects of absorbency

$$Absorption\ ability\ =\ (W2\ -\ W1)/W1$$

(W1: sample weight and W2: measured 30 minutes after reaction with saline solution)

(2) Test results

[Table 2]

| Classification | Absorption ability 1 | Absorption ability 2 | Absorption ability 3 | Average |
|---|---|---|---|---|
| Example 1 | 31.2 | 31.8 | 35.8 | 32.9 |
| Comparative Example 1 | 7.5 | 7.7 | 7.3 | 7.5 |
| Comparative Example 2 | 15.5 | 16.2 | 15.9 | 15.9 |

As can be seen in Table 2, the medical dressing material having the chitosan fabric sponge structure of Example 1 exhibits excellent absorption ability compared to Comparative Examples 1 and 2.

2-2) Evaluation of blood clotting

(1) Test method

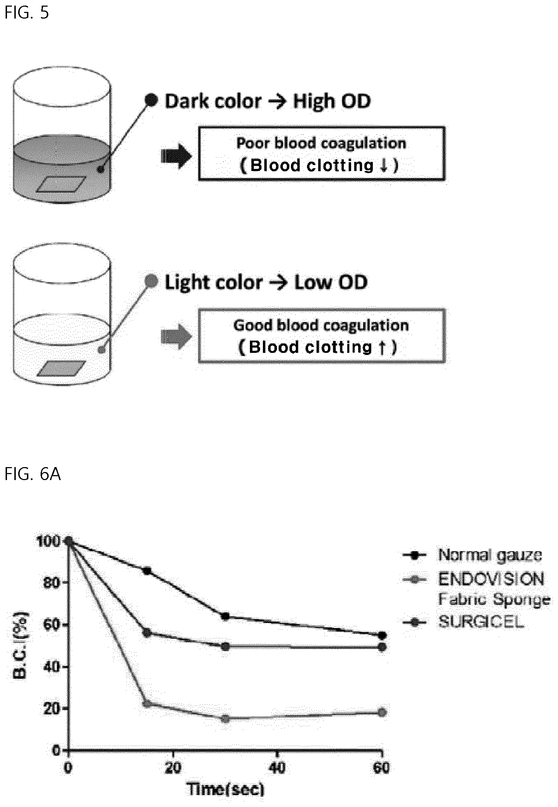

As can be seen in the following FIG. 5, blood was collected from NZW rabbit veins and was then brought into contact with the sample, so that an O.D. value was measured at 542 nm to calculate a blood-clotting index (BCI) . When blood clotting occurs well, the color of the blood becomes lighter and the O.D. is low. When blood clotting does not occur well, the color of the blood is not changed relatively and the O.D. is high.

$$Blood-clotting\ index\ (BCI)\ =\ (OD_t/OD_b)\ *\ 100$$

($OD_t$: average absorbance of experimental group, $OD_b$: average absorbance of blank)

(2) Test results

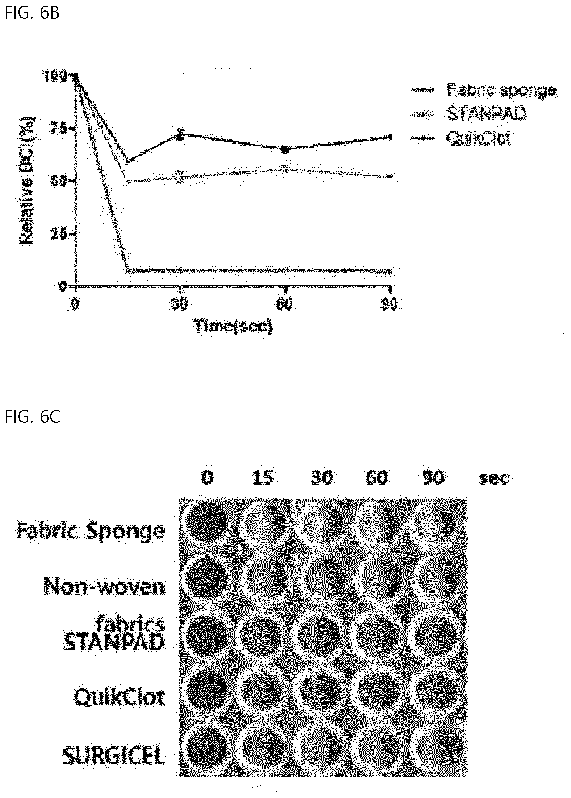

As shown in the following FIGS. 6A to 6C, the medical dressing material having the chitosan fabric sponge structure of Example 1 exhibits a fast absorption rate and does not again release blood after blood absorption, thus having excellent blood coagulation power compared to Comparative Example 1 (normal gauze/non-woven fabrics), Comparative Example 3 (SURGICEL), Comparative Example 4 (STANPAD), and Comparative Example 5 (QuicClot).

2-3) Evaluation of blood absorption rate

(1) Test method

The time (sec.) required for the sample, cut to a size of 3 x 3 cm, to absorb the maximum amount of blood was measured.

(2) Test results

[Table 3]

| Classification | Absorption rate 1 (sec.) | Absorption rate 2 | Absorption rate 3 | Average |
|---|---|---|---|---|
| Example 1 | 7 | 6 | 7 | 6.7 |
| Comparative Example 1 | 11 | 10 | 10 | 10.3 |

(continued)

| Classification | Absorption rate 1 (sec.) | Absorption rate 2 | Absorption rate 3 | Average |
|---|---|---|---|---|
| Comparative Example 2 | 8 | 8 | 7 | 7.7 |

As can be seen in Table 3, the medical dressing material having the chitosan fabric sponge structure of Example 1 exhibits an excellent blood absorption rate compared to Comparative Examples 1 and 2.

2-4) Evaluation of deodorization rate

(1) Test method

An ammonia gas detector tube method was used. That is, the level of ammonia remaining in a plastic bag was measured 2 hours after the sample was put into the plastic bag filled with ammonia gas, and a deodorization rate was calculated using the following Equation.

$$\text{Deodorization rate (\%)} = ((C_b - C_s)/C_b) * 100$$

($C_b$: BLANK, concentration of the test gas remaining in the test gas bag after 2 hours, and $C_s$: Sample, concentration of the test gas remaining in the test gas bag after 2 hours)

(2) Test result

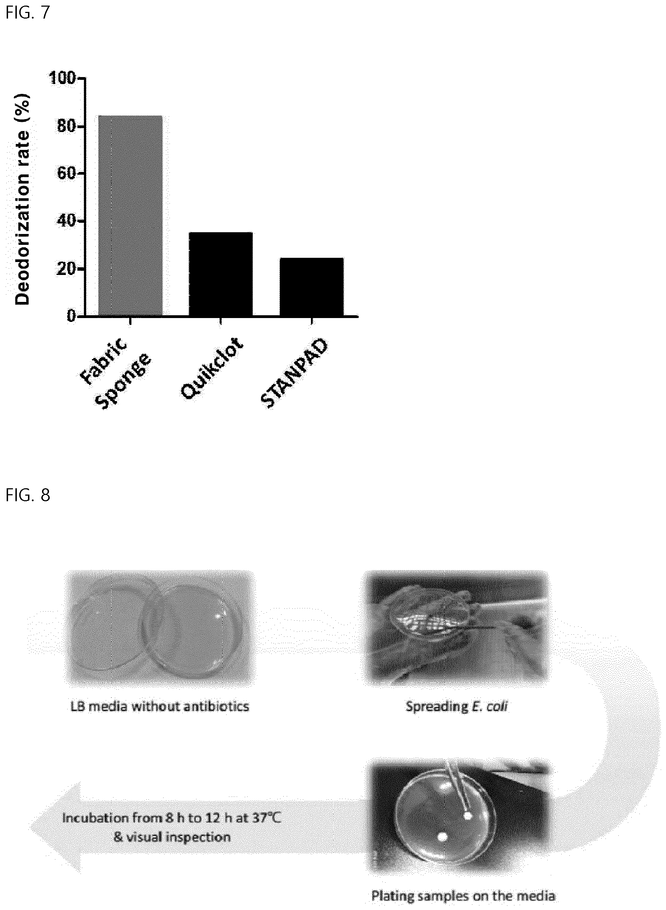

As can be seen in the following FIG. 7, the medical dressing material having the chitosan fabric sponge structure of Example 1 effectively deodorizes ammonia generated during the decomposition of proteins, thereby exhibiting an excellent deodorization rate compared to Comparative Example 4 (STANPAD) and Comparative Example 5 (QuicClot).

2-5) Evaluation of ability to withstand moisture

(1) Test method

The tensile strength was measured 60 seconds after immersion in water at 37°C.

(2) Test result

[Table 4]

| Classification | Tensile strength 1 (unit: gf) | Tensile strength 2 | Tensile strength 3 | Average |
|---|---|---|---|---|
| Example 1 | 1480 | 1420 | 1470 | 1456.7 |
| Comparative Example 1 | 800 | 950 | 840 | 863.3 |
| Comparative Example 2 | Not measurable | Not measurable | Not measurable | Not measurable |

In the case of Comparative Example 2, for example, the shape may be easily collapsed after moisture absorption, so it was impossible to measure tensile strength using a tensile strength meter. As can be seen in Table 4, the medical dressing material having the chitosan fabric sponge structure of Example 1 had excellent ability to withstand moisture compared to Comparative Examples 1 and 2.

2-6) Evaluation of ability to withstand friction

Humidification was performed at a constant pressure. After 6 hours, physical friction was applied three times at the same pressure, and a visual evaluation was then performed. In the visual evaluation, the degree of damage to the product, such as the occurrence of debris and fragments, was comprehensively evaluated after the physical friction, and the level was divided into ten stages, ranging from a low effect (1) to a high effect (10).

[Table 5]

| Classification | Level 1 (unit: score) | Level 2 | Level 3 | Average |
|---|---|---|---|---|
| Example 1 | 9 | 8 | 10 | 9 |
| Comparative Example 1 | 5 | 5 | 6 | 5.3 |
| Comparative Example 2 | 2 | 2 | 2 | 2 |

As can be seen in FIG. 5, the medical dressing material having the chitosan fabric sponge structure of Example 1 has excellent ability to withstand friction compared to Comparative Examples 1 and 2.

2-7) Evaluation of antibacterial effect

(1) Test method

As shown in the following FIG. 8, *E. coli* was mixed with the sample and incubated at 37°C for 8 to 12 hours.

(2) Test result

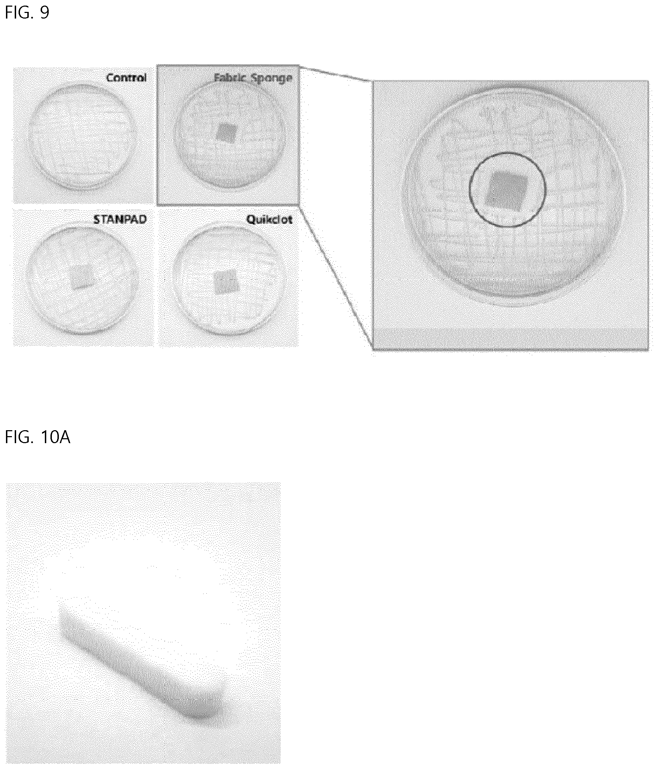

As shown in the following FIG. 9, in the case of the medical dressing material having the chitosan fabric sponge structure of Example 1 (fabric sponge), the proliferation of *E. coli* is suppressed in the area in contact with the fabric sponge (red circle), thereby securing an excellent antibacterial effect, compared to Comparative Example 4 (STANPAD) and Comparative Example 5 (QuicClot).

Experimental Example 3

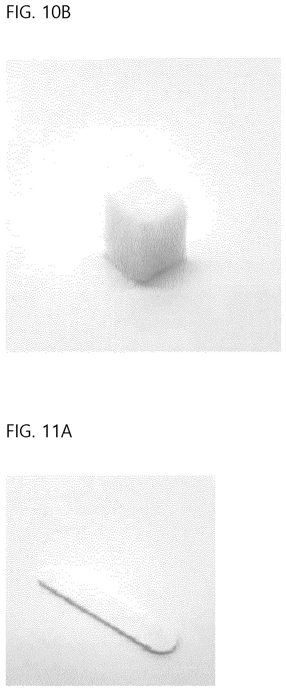

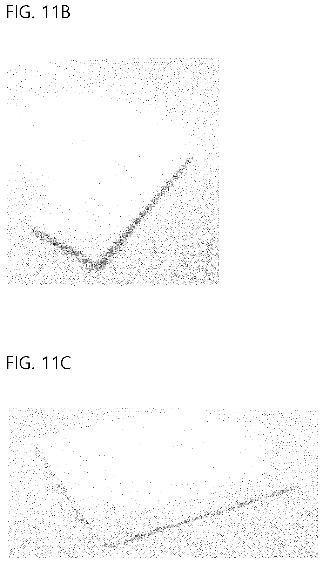

Photographs of the commercialized medical dressing material having the chitosan fabric sponge structure manufactured according to Example 1 are shown in the following FIGS. 10A and 10B. FIG. 11 shows an image in which FIG. 10B is applied to the oral cavity.
Such a medical dressing material may be used for various body parts. For example, the dressing material of FIG. 10B may be suitably used for the oral cavity after tooth extraction or for managing gum bleeding.

Experimental Example 4

Photographs of the commercialized medical dressing material having the chitosan fabric sponge structure manufactured using a process including compression according to Example 2 are shown in the following FIGS. 12A to 12C. FIG. 13A shows an image in which FIG. 12A is applied to the nasal cavity, and FIG. 13B shows an image in which FIG. 12B is applied to the oral cavity.
Such a medical dressing material may be used for various body parts. For example, the dressing material of FIG. 12A may be used for the nasal cavity, such as management of bleeding after nasal surgery, and the dressing material of FIG. 12B may be suitably used for the oral cavity, such as management of gum bleeding. Further, the dressing material of FIG. 12C may be suitably used for acute and chronic wounds by an edged thing.

**Claims**

1. A method of manufacturing a medical dressing material having a chitosan fabric sponge structure, comprising:

   (A) preparing a chitosan fabric;
   (B) immersing the chitosan fabric in an acidic organic solution, thus performing acid treatment;
   (C) washing the acid-treated chitosan fabric using alcohol;
   (D) agitating a gel obtained by mixing the washed chitosan fabric with an aqueous solvent, thus performing homogenization; and
   (E) freeze-drying a homogenized chitosan fabric gel to manufacture a chitosan fabric sponge.

2. The method of claim 1, wherein in step (B), the acid treatment is performed for 1 to 10 minutes.

3. The method of claim 1, wherein in step (B), an acidic compound and an alcohol solvent are mixed at a ratio of 1 : 99 to 7 : 93 in the acidic organic solution based on a weight thereof.

4. The method of claim 3, wherein the acidic compound is one or more selected from the group consisting of hydrochloric acid, acetic acid, ascorbic acid, nitric acid, sulfuric acid, hydrofluoric acid, and phosphoric acid.

5. The method of claim 3, wherein the alcohol solvent is one or more selected from the group consisting of methanol, ethanol, propanol, and butanol.

6. The method of claim 1, wherein in step (C), the washing includes a first washing step, a second washing step, and a third washing step continuously performed using 100% alcohol.

7. The method of claim 6, wherein each of the first washing step, the second washing step, and the third washing step is performed for 20 to 90 seconds.

8. The method of claim 1, wherein in step (D), the aqueous solvent is distilled water or ultrapure water.

9. The method of claim 1, wherein in step (D), the gel is obtained by mixing 1 to 5 parts by weight of the washed chitosan fabric based on 100 parts by weight of the aqueous solvent.

10. The method of claim 1, wherein in step (D), the homogenization is performed at 5,500 to 20,000 rpm for 1 to 10 minutes using a homogenizer.

11. The method of claim 1, wherein step (E) includes:

    (E-1) injecting the homogenized chitosan fabric gel into a molding frame; and
    (E-2) putting the molding frame into a freeze dryer to perform freeze-drying, thus manufacturing the chitosan fabric sponge.

12. The method of claim 11, wherein in step (E-2), the freeze-drying is performed while a temperature is increased from a first temperature ($\Delta t_1$) to a second temperature ($\Delta t_2$) for 2.5 to 5.5 days, the first temperature ($\Delta t_1$) is -40 to -50°C, and the second temperature ($\Delta t_2$) is 40 to 50°C.

13. The method of claim 11, wherein a preliminary freezing step is performed between steps (E-1) and (E-2), and the preliminary freezing step includes:

    (E-1a) putting the molding frame into the freeze dryer and storing the molding frame at 3 to 5°C for 450 to 550 minutes to thus remove air bubbles, followed by cooling to 0°C;
    (E-1b) maintaining a temperature for a first time ($\Delta m_1$) after step (E-1a);
    (E-1c) reducing the temperature by $\Delta T$ for a second time ($\Delta m_2$) after step (E-1b); and
    (E-1d) repeating the step (E-1b) and step (E-1c) until the temperature becomes -40 to -50°C after the step (E-1c), and then maintaining the temperature for 100 to 140 minutes.

14. The method of claim 13, wherein each of the first time ($\Delta m_1$) and the second time ($\Delta m_2$) is 20 to 70 minutes, and $\Delta T$ is 5 to 10°C.

15. The method of claim 1, further comprising:
    (F) performing thermal compression on the freeze-dried chitosan fabric sponge.

16. The method of claim 1 or 15, further comprising:
    (G) adding at least one X-ray sensitive material so as to identify a location of the medical dressing material using an X-ray device.

17. A medical dressing material having a chitosan fabric sponge structure in which chitosan macro fibers and chitosan micro fibers derived from a chitosan fabric are located on a porous structure formed using freeze-drying.

18. The medical dressing material of claim 17, wherein the medical dressing material has a density of 0.01 to 0.1 g/cm$^3$.

19. The medical dressing material of claim 17, wherein the medical dressing material has a wet tensile strength of 500 to 1500 gf and a dry tensile strength of 1000 to 2000 gf.

20. The medical dressing material of claim 17, wherein the medical dressing material includes at least one X-ray sensitive material so as to identify a location of the dressing material using an X-ray device.

21. The medical dressing material of claim 17, wherein the medical dressing material includes a hemostatic dressing material and a dressing material for wounds by an edged thing.

FIG. 1A

FIG. 1B

Chitosan fiber

FIG. 2A

FIG. 2B

FIG. 3A

FIG. 3B

FIG. 4

FIG. 5

FIG. 6A

FIG. 6B

FIG. 6C

FIG. 7

FIG. 8

LB media without antibiotics

Spreading *E. coli*

Plating samples on the media

Incubation from 8 h to 12 h at 37°C & visual inspection

FIG. 9

FIG. 10A

FIG. 10B

FIG. 11A

FIG. 11B

FIG. 11C

# EP 3 782 656 A1

## INTERNATIONAL SEARCH REPORT

| International application No. |
|---|
| PCT/KR2019/004614 |

**A. CLASSIFICATION OF SUBJECT MATTER**

*A61L 15/42(2006.01)i, A61L 15/28(2006.01)i, A61F 13/00(2006.01)i, A61F 13/36(2006.01)i, C08L 5/08(2006.01)i, A61L 15/54(2006.01)i*

According to International Patent Classification (IPC) or to both national classification and IPC

**B. FIELDS SEARCHED**

Minimum documentation searched (classification system followed by classification symbols)

A61L 15/42; A61F 13/15; A61K 31/715; A61K 31/78; A61L 15/28; A61L 15/44; A61L 27/60; A61L 31/04; C08J 3/075; A61F 13/00; A61F 13/36; C08L 5/08; A61L 15/54

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: chitosan, fiber, sponge, freeze-drying, hemostasis and dressing

**C. DOCUMENTS CONSIDERED TO BE RELEVANT**

| Category* | Citation of document, with indication, where appropriate, of the relevant passages | Relevant to claim No. |
|---|---|---|
| X | US 2009-0226391 A1 (ROBERTS, Keith A. et al.) 10 September 2009 See paragraphs [0013]-[0048]; claim 1; and figures 1-5. | 17-19,21 |
| Y | | 20 |
| A | | 1-16 |
| Y | KR 10-1700107 B1 (ENDO VISION CO., LTD. et al.) 26 January 2017 See paragraph [0053]; claim 1; and figure 2. | 20 |
| A | KR 10-2017-0047168 A (KOO, Tae Hoon) 04 May 2017 See paragraphs [0034]-[0050], [0070]; claims 1-11; and figure 2. | 1-21 |
| A | KR 10-2017-0123292 A (KOO, Tae Hoon) 07 November 2017 See paragraphs [0024]-[0057] and figures 1-2. | 1-21 |
| A | KR 10-2004-0090033 A (HYOSUNG CORPORATION) 22 October 2004 See pages 3-5. | 1-21 |

☐ Further documents are listed in the continuation of Box C.   ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 11 JULY 2019 (11.07.2019) | **12 JULY 2019 (12.07.2019)** |

| Name and mailing address of the ISA/KR | Authorized officer |
|---|---|
| Korean Intellectual Property Office Government Complex Daejeon Building 4, 189, Cheongsa-ro, Seo-gu, Daejeon, 35208, Republic of Korea Facsimile No. +82-42-481-8578 | Telephone No. |

Form PCT/ISA/210 (second sheet) (January 2015)

**INTERNATIONAL SEARCH REPORT**
Information on patent family members

International application No.

**PCT/KR2019/004614**

| Patent document cited in search report | Publication date | Patent family member | Publication date |
|---|---|---|---|
| US 2009-0226391 A1 | 10/09/2009 | US 9061087 B2<br>WO 2009-111282 A2<br>WO 2009-111282 A3 | 23/06/2015<br>11/09/2009<br>22/07/2010 |
| KR 10-1700107 B1 | 26/01/2017 | None | |
| KR 10-2017-0047168 A | 04/05/2017 | KR 10-1887761 B1 | 10/08/2018 |
| KR 10-2017-0123292 A | 07/11/2017 | KR 10-1849843 B1 | 18/04/2018 |
| KR 10-2004-0090033 A | 22/10/2004 | KR 10-0608192 B1 | 02/08/2006 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20010047248

- KR 0644369